(19) **Europäisches Patentamt**

**European Patent Office**

**Office européen des brevets**

(11) Veröffentlichungsnummer : **0 502 919 B1**

(12) # EUROPÄISCHE PATENTSCHRIFT

(45) Veröffentlichungstag der Patentschrift :
**03.11.93 Patentblatt 93/44**

(51) Int. Cl.$^5$ : **A61N 1/00,** A61N 2/04

(21) Anmeldenummer : **90917746.1**

(22) Anmeldetag : **03.12.90**

(86) Internationale Anmeldenummer :
**PCT/DE90/00932**

(87) Internationale Veröffentlichungsnummer :
**WO 91/08015 13.06.91 Gazette 91/13**

(54) **GERÄT ZUR HIRNWELLENSTIMULATION.**

(30) Priorität : **02.12.89 DE 3939974**

(43) Veröffentlichungstag der Anmeldung :
**16.09.92 Patentblatt 92/38**

(45) Bekanntmachung des Hinweises auf die
Patenterteilung :
**03.11.93 Patentblatt 93/44**

(84) Benannte Vertragsstaaten :
**AT CH DE ES FR GB IT LI**

(56) Entgegenhaltungen :
**EP-A- 0 076 125
EP-A- 0 290 126
WO-A-89/04191
US-A- 4 195 626**

(56) Entgegenhaltungen :
**US-A- 4 556 069
US-A- 4 861 154
US-A- 4 875 484
DERWENT'S ABSTRACT, No. 89-98 307/13,
publ. week 8913, & SU, A, 1414312 (NEMEK-
TRON GmbH)**

(73) Patentinhaber : **WUNSCH, Alexander
Bergheimer Strasse 116
D-69115 Heidelberg (DE)**

(72) Erfinder : **WUNSCH, Alexander
Bergheimer Strasse 116
D-69115 Heidelberg (DE)**

(74) Vertreter : **Naumann, Ulrich et al
Patentanwälte, Ullrich & Naumann,
Gaisbergstrasse 3
D-69115 Heidelberg (DE)**

EP 0 502 919 B1

**Beschreibung**

Die Erfindung betrifft ein Gerät zur Hirnwellenstimulation mit einer Einrichtung zur Einstellung einer Frequenz und mit Impulsformungs- und Aufbereitungseinheiten zur Ausgabe akustischer, optischer, elektrischer und/oder elektromagnetischer bzw. mechanischer Impulssignale.

Bei den Hirnwellenbereichen unterscheidet man

```
Delta-Wellen    0,5 -   4 Hz   im Tiefschlaf,

Theta-Wellen    4   -   8 Hz   bei der Tiefenentspannung
                               und im REM-Schlaf,

Alpha-Wellen    8   - 16 Hz    in Ruhe- und Entspannungs-
                               phasen bei geschlossenen
                               Augen,

Beta-Wellen     16  - 32 Hz    im Wachzustand, Stress, und

Gamma-Wellen    32  - 64 Hz    (bis in den Kilohertzbereich
                               vorkommend).
```

Bei der Hirnwellenstimulation werden Impulse in den obengenannten Bereichen entweder als
Lichtimpulse,
Tonimpulse,
elektrische Impulse und/oder
elektromagnetische Impulse
durch entsprechende Geräte appliziert. Das Gehirn schwingt sich aufgrund der Frequenz-Folge-Reaktion nach einiger Zeit auf die applizierte Frequenz ein. Damit stellen sich auch die für diese Frequenz spezifischen Gehirnfunktionen ein.

Durch wiederholte Anwendung tritt ein Lernprozeß ein, der es dem Patienten ermöglicht, gewünschte Hirnwellenbereiche leichter aufzusuchen.

Es sind aus der Praxis Geräte bekannt, die mit Licht- und Tonimpulsen, mit elektrischen Impulsen zur sogenannten transkutanen elektrischen Nervenstimulation (TENS-Geräte), sowie mit elektromagnetischen Impulsen (Magnetfeldtherapiegeräte) arbeiten.

Sowohl aus der US-A 4 195 626 als auch aus der WO-A 89/04191 sind Geräte zur Hirnwellenstimulation mit einer Einrichtung zur Einstellung einer Frequenz und mit Impulsformungs- und Aufbereitungseinheiten zur Ausgabe akustischer, optischer, elektrischer und/oder elektromagnetischer bzw. mechanischer Impulssignale bekannt. Die US-A 4 195 626 beschreibt insbesondere einen Mikroprozessor gesteuerten Stimulator, dessen Taktfrequenz von einem Kontrolloszillator mit programierbaren Frequenzteilern hergeleitet wird. Die WO-A 89/04191 offenbart keine detaillierte Schaltung.

Des weiteren ist aus der Literaturstelle "Derwent's Abstract, Nr. 89-98 307/13 Publ. Woche 8913, & SU-A-1 414 312" ein Indifferenzstromgenerator bekannt, bei dem es im wesentlichen darauf ankommt, eine bestimmte Frequenzdifferenz der Ausgangssignale zu erzielen. Die Ausgangssignale werden dabei über zwei unterschiedliche Frequenzteiler aus einem gemeinsamen Oszillator hergeleitet. Schließlich ist aus der US-A 4 556 069 eine Testmethode sowie ein entsprechendes Testgerät für das menschliche Gehör bekannt, wobei hier keine Frequenzabstimmung erfolgt.

Die Nachteile der bekannten Geräte liegen in ihrer unzureichenden Einstellbarkeit der Frequenz hinsichtlich der Frquenzstabilität, Frequenzwiederholbarkeit und freien Einstellbarkeit in weiten Frequenzbereichen. Sie sind ferner unhandlich und arbeiten mit Brillen bzw. Helmen, die für den Anwender unbequem und meist als störend empfunden werden.

Der Erfindung liegt die Aufgabe zugrunde, ein Gerät der eingangs genannten Art zu schaffen, das die Erzeugung von Licht-, Ton-, elektrischen und/oder elektromagnetischen Impulsen zur Applikation am Anwender mit hoher Frequenzstabilität bei freier Wählbarkeit einer gewünschten Frequenz in Stufen im 100-stel Hertz-Bereich gestattet, dabei aber so ausgeführt ist, daß es für den Anwender bequem zu tragen, möglichst wenig störend und einfach in der Handhabung ist.

Diese Aufgabe wird mit einem Gerät nach den Merkmalen des Patentanspruches 1 gelöst. Danach ist ein Gerät der eingangs genannten Art so ausgestaltet, daß einem vorzugsweise quarzstabilisierten Schwingkreis zur Frequenzeinstellung ein digitaler Frequenzteilerbaustein nachgeschaltet ist, der über einen Umschalter mit einem programmierbaren digitalen Frequenzteiler verbunden ist, daß der Frequenzteiler eine definierte Frequenzeinstellung in Schritten bis zu 0,01 Hertz ermöglicht, daß dem Frequenzteiler zwei weitere digitale Frequenzteiler nachgeschaltet sind, daß die Frequenzteiler in Reihe geschaltet sind und je zwei Ausgänge aufweisen, daß die Ausgänge über gekoppelte Umschalter unterschiedliche Frequenzen an Impulsformungseinheiten für verschiedene Applikationsarten liefern und daß die Impulsformungseinheiten für angeschlossene Applikatoren Ausgangssignale erzeugen, die identische Frequenz aber unterschiedliche Impulsformen aufweisen.

Vorteilhafte Weiterbildungen des erfindungsgemäßen Gerätes sind durch die Merkmale der Unteransprüche vorgegeben.

Zu den wesentlichen Bauteilen des erfindungsgemäßen Gerätes gehört ein Frequenzgenerator (Einrichtung zur Einstellung einer Frequenz) mit Quarzstabilisierung zur Erzeugung einer invariablen Frequenz im Megahertz-Bereich, wobei diese Frequenz durch eine digitale Frequenzteilereinheit geteilt wird, die wiederum mittels einer Eingabeeinheit auf ein definiertes Teilerverhältnis programmiert werden kann. Der erhaltene Impuls definierter Frequenz wird an eine Inverterschaltung geleitet, die zudem die Rechteck-Wellen-Charakteristik der Impulse verbessert. Mit den aufbereiteten invertierten und nicht-invertierten Rechteck-Impulsen werden elektronische Schalter gesteuert, die den jeweiligen Applikator betätigen.

Das erfindungsgemäße Gerät kann in vorteilhafter Weise so ausgelegt werden, daß eine direkte Frequenzeingabe in Ablesung mittels dezimaler Tast-Codierschalter erfolgt, die mit wechselndem Dezimalpunkt für verschiedene Frequenzbereiche versehen sind.

Ein weiterer besonderer Vorteil des erfindungsgemäßen Geräts besteht darin, daß damit ein kleines, handliches, leicht bedienbares Gerät geschaffen ist.

Des weiteren kann der mit dem Gerät verwendete opto-akustische Impulsapplikator ein einfacher, handelsüblicher Bügel-Kopfhörer mit Hörmuscheln und daran schwenkbar angeordneten Halbbügeln bzw. Bügelarmen sein, die für je eine Lichtquelle vorgesehen sind. An den einander zugekehrten Enden der Bügelarme kann je eine Glühbirne oder eine Leuchtdiode angeordnet sein. Die Lichtquellen können mit den schwenkbaren Bügelarmen leicht in Augenhöhe des Anwenders gedreht und ensprechend dem Augenabstand eingerichtet werden. Ein solcher Bügel-Kopfhörer hat nur ein geringes Gewicht, ist bequem zu tragen und wird vom Anwender als wenig störend empfunden. Als "Ambulanzgerät" kann es vom Patienten bzw. Anwender selbst beispielsweise durch Miniatur-Dreh-Codierschalter bedient und eingestellt werden. Es arbeitet bei geringer Leistungsaufnahme mit Batteriebetrieb.

In besonders vorteilhafter Weise läßt sich ein als elektromagnetischer Wandler ausgeführter Applikator so ausgestalten und anordnen, daß der elektromagnetische Wandler ein auf den Anwender zur Einwirkung bringbares Magnetfeld definierter Frequenzen erzeugt. Der elektromagnetische Wandler kann des weiteren einen Wickelkörper aus Kunststoff und zwei darauf gewickelte, parallel laufende Drähte aufweisen. Die daraus resultierenden Spulen können des weiteren gegenläufig betrieben werden, so daß sich die von den Spulen ausgehenden Magnetfelder im wesentlichen aufheben. Sich selbst aufhebenden Magnetfeldern werden besondere biologische Wirkungen zugeschrieben.

Da fast alle Gehirnleistungen mit bestimmten Gehirnwellenfrequenzen assoziiert sind, ist das Anwendungsspektrum des erfindungsgemäßen Gerätes groß. Anwendungen empfehlen sich beispielsweise bei Schmerzzuständen, Schlafstörungen, psychischen Explorationen, Streßzuständen, Muskelverspannungen, Antriebsarmut, Drogenabhängigkeit, Depressionen, Störungen der hormonalen Homöostate, etc. Des weiteren läßt sich das Gerät in vorteilhafter Weise zur Lernverbesserung (Superlearning), zur Reaktionsbeschleunigung im Sport sowie zur Unterstützung von Heilungsprozessen einsetzen.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung von Ausführungsbeispielen der Erfindung anhand der Zeichnung zu verweisen. In Verbindung mit der Erläuterung der bevorzugten Ausführungsbeispiele der Erfindung anhand der Zeichnung werden auch im allgemeinen bevorzugte Ausgestaltungen und Weiterbildungen der Lehre erläutert. In der Zeichnung zeigt

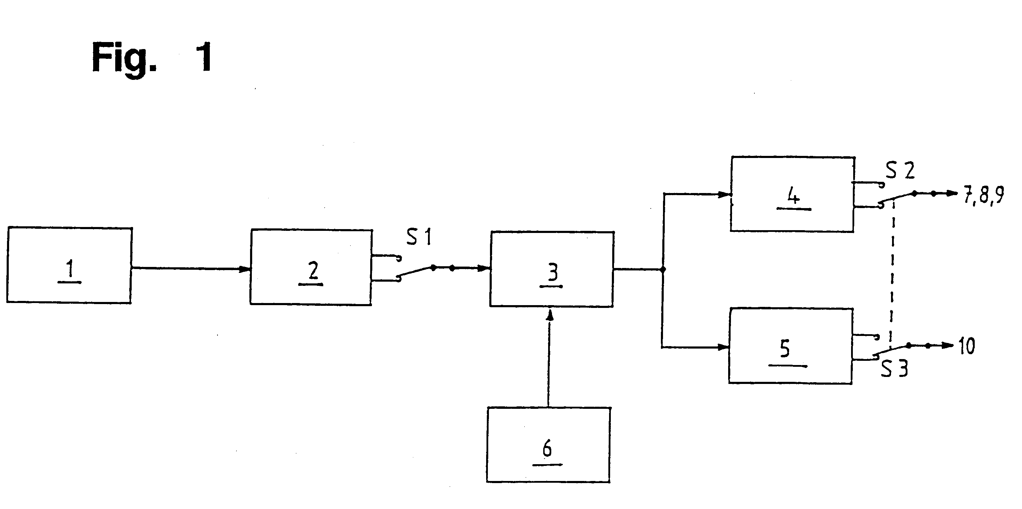

Fig. 1         in einem Blockschaltbild, schematisch, den Aufbeu eines Ausführungsbeispiels des erfindungsgemäßen Gerätes,

| Fig. 2 | in einem Blockschaltbild, schematisch, die Anordnung von Impulsformungs- und Aufbereitungseinheiten eines Ausführungsbeispiels des erfindungsgemäßen Gerätes, |
| Fig. 3 | in einem Blockschaltbild, schematisch, den Aufbau eines Schieberegisters eines Ausführungsbeispiels des erfindungsgemäßen Gerätes, |
| Fig. 4 | in schematischer Darstellung einen optoakustischen Impulsapplikator, |
| Fig. 5 | in schematischer Darstellung einen als Elektrode ausgeführten Impulsapplikator, |
| Fig. 6 | in schematischer Darstellung einen Sägezahnimpuls-Stecker zur Verbindung der Elektroden mit dem erfindungsgemäßen Gerät, |
| Fig. 7 bis 11 | in Diagrammen die Beeinflussung der Impulsform in Abhängigkeit von der Frequenz, |
| Fig. 12 | in schematischer Darstellung ein aus zwei Opto-Kopplern bestehendes Modul zur Ansteuerung des Tonimpuls-Applikators, |
| Fig. 13 | in schematischer Darstellung den Aufbau eines Opto-Kopplers des in Fig. 12 gezeigten Moduls und |
| Fig. 14 | in einem Blockschaltbild, schematisch, eine Fotonik-Schaltung zur Ansteuerung einer Fotonik-Folie des Lichtimpuls-Applikators. |

Im quarzstabilisierten Schwingkreis 1 ist ein Quarz verwendet, der eine sehr stabile, genau definierte Frequenz im MegahertzBereich erzeugt. Anschlossen an den Schwingkreis 1 ist ein Frequenzteilerbaustein 2 mit zwei Ausgängen, die über einen Umschalter S1 an einen programmierbaren digitalen Frequenzteiler 3 gelangen. Der Umschalter S1 ermöglicht die Wahl des Frequenzbereiches. Dem Frequenzteiler 3 ist eine Eingabeeinheit 6 zugeordnet, durch die eine definierte Frequenz in Schritten von bis zu 0,01 Hertz eingestellt werden kann. Zweckmäßigerweise sind mehrere (nicht dargestellte) Eingabeeinheiten vorgesehen, um über Umschalter bereits voreingestellte Frequenzen abrufen zu können. Solche (nicht dargestellten) Umschalter können beispielsweise als elektronische Schalter ausgeführt sein, die durch ein Schieberegister betätigt werden, wobei ein Triggerimpuls die Weiterschaltung auslöst. Die Anzahl der Registerplätze und der Eingabeeinheiten sind dabei identisch. Der Triggerimpuls kann durch mechanische Betätigung eines Tastschalters oder durch einen Schwellenwertschalter erzeugt werden, der beispielsweise bei Erreichen eines bestimmten Widerstandswertes einen Impuls abgibt.

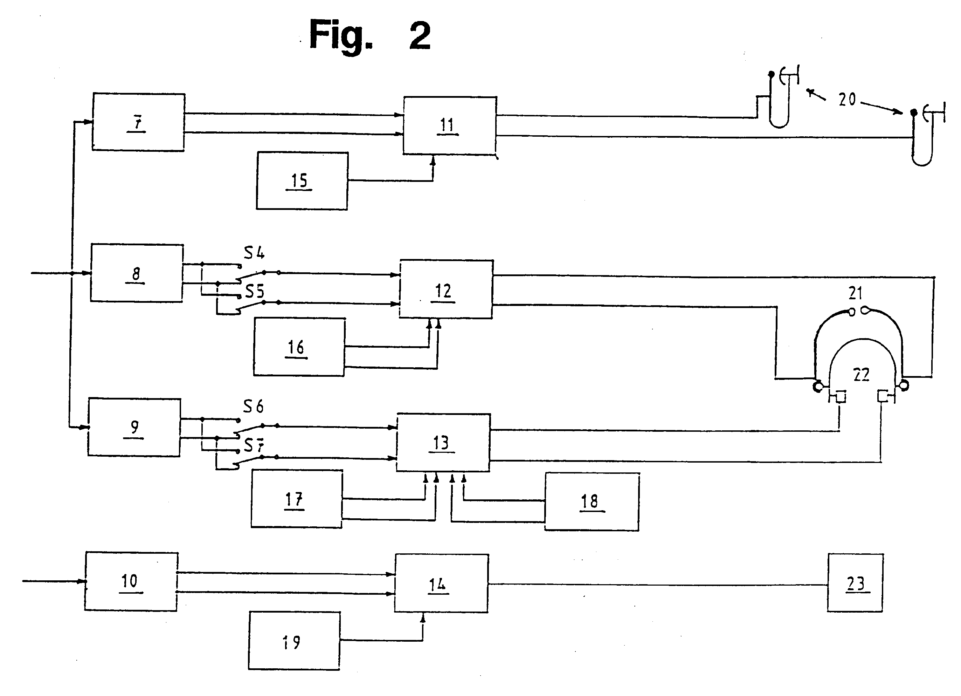

Dem Frequenzteiler 3 sind zwei parallel zueinander angeordnete digitale Frequenzteiler 4 und 5 nachgeschaltet. Jeder dieser Frequenzteiler 4 und 5 weist zwei Ausgänge auf, die über gekoppelte Umschalter S2 bzw. S3 eine weitere Frequenzbereichsumschaltung ermöglichen und gemäß der Darstellung in Fig. 2 zu Impulsformungseinheiten 7, 8, 9 bzw. 10 führen. Hiermit wird eine Anpassung an diese Impulsformungseinheiten erreicht. So ist im hier gewählten Ausführungsbeispiel die Frequenz am Eingang der Impulsformungseinheiten 7, 8 und 9 um den Faktor 100 höher als die Frequenz am Eingang der Impulsformungseinheit 10.

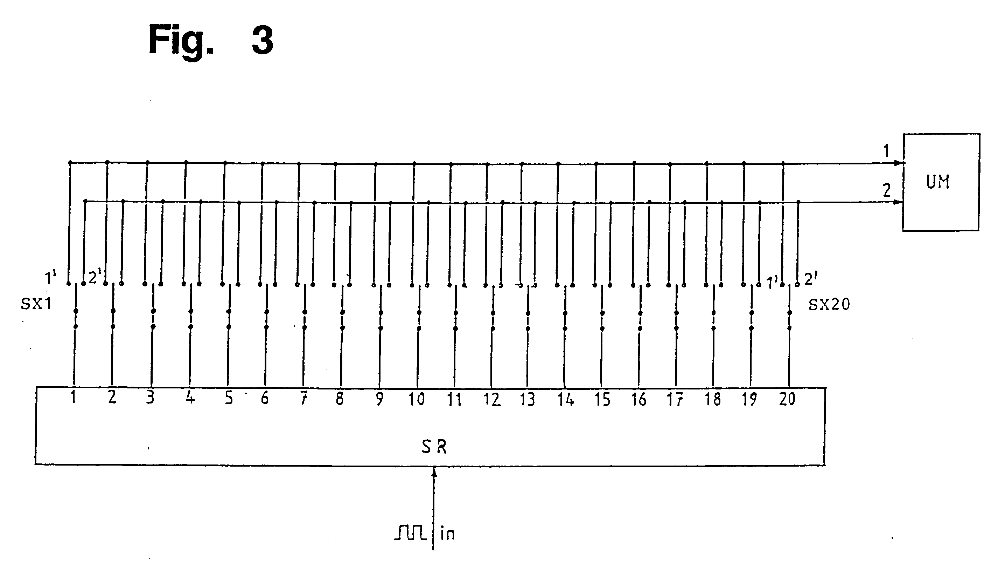

Die Impulsformungseinheit 7 weist einen weiteren Frequenzteiler auf, dessen Teilungsverhältnis T sich aus dem Quotienten 100/N ergibt, wobei N gleich der Anzahl der Registerplätze SX1 - SXn eines Schieberegisters SR ist (vgl. Fig. 3). Für ein 20-stelliges Schieberegister ergibt sich dabei ein Teilerverhältnis T = 5. Jeder im Schieberegister SR eintreffende Impuls verschiebt den aktiven Ausgang um eine Stelle auf den nachfolgenden Schalter mit mittlerer Nullstellung.

Der Impulsformungseinheit 7 ist eine aus elektronischen Schaltern realisierte Umpolungsschaltung 11 (entspricht UM in Fig. 3) nachgeschaltet, der eine einstellbare Stromquelle 15 zugeordnet ist. Die Umpolungsschaltung 11 schaltet den aus der Stromquelle gelieferten Konstantstrom oder widerstandsabhängigen Strom in den Anwenderstromkreis, d.h. in Applikatoren.

Gemäß der Darstellung in Fig. 3 ist die Impulsformungseinheit als Schieberegister SR ausgeführt. Mit dem Schieberegister SR nachgeschalteten Schaltern SX1 bis SX20 mit mittlerer Nullstellung wird die Hüllkurve des an den Anwender gelangenden Stromes in der Art definiert, so daß bei Schalterstellung 1' der Anwenderstrom in die eine Richtung und bei Schalter 2' in die entgegengesetzte Richtung fließt. Bei Mittelstellung fließt kein Strom. Hierdurch wird eine Änderung der Stromflußrichtung ohne die Definition eines künstlichen Massepunktes erreicht. Durch die Impulsformungseinheit 7 bzw. das Schieberegister SR findet für den Fall eines 20-stelligen Schieberegisters SR eine Zusammensetzung des Ausgangssignals in 5%-Schritten statt. Durch die Teilung des Signals durch den Faktor 100 (N x T), erscheint an dem dem Anwenderstromkreis angeschlossnen Elektroden-Applikator 20 (Fig. 2) ein Impuls derselben Frequenz, wie sie am Eingang der Impulsformungseinheit 10 erscheint, zumal die Impulsformungseinheit 10 sowie die dort nachgeschaltete Elektronik die in die Impulsformungseinheit 10 eingehende Frequenz nicht moduliert. Diese Frequenz ist identisch mit den Frequenzen an den an den Impulsformungseinheiten 8, 9 und 10 angeschlossenen Applikatoren 21, 22 und 23, deren Anschluß noch beschrieben wird.

Die Impulsformungseinheiten 8 und 9 sind im wesentlichen identisch. Durch zwei Dezimalzähler, die als "Teiler-durch-Zehn" geschaltet sind, wird das Eingangssignal durch den Faktor 100 geteilt, wobei das Tastverhältnis in 1%-Schritten zwischen 1% und 99% mittels Zweitast-Codierschaltern einstellbar ist. Durch zwei nachgeschaltete Schmitt-Trigger-Invertierschaltungen wird die Flankensteilheit des Pulsbreiten-modulierten Signals verbessert und ein invertiertes und ein nicht-invertiertes Signal werden auf die Eingänge der Umschalter S4 und S5 bzw. S6 und S7 gegeben.

Die Impulformungseinheit 8 ist über die Umschalter S4 und S5 mit einem elektronischen Schalter 12 verbunden, dem eine Lichtverstärkerschaltung 16 zugeordnet ist. Diese wird über den durch die Impulsformungseinheit 7 getakteten elektrischen Schalter auf die Lichtimpuls-Ausgabeeinheit 21 geschaltet. Dabei kann eine Regelung der Grundhelligkeit sowie der Lichtimpulshelligkeit vorgenommen werden.

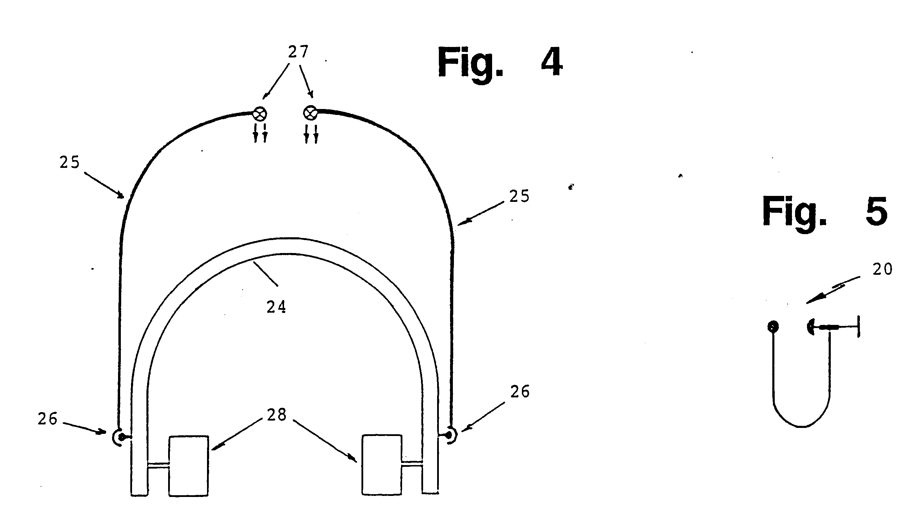

In gleicher Weise arbeitet die Impulformungseinheit 9. Diese führt über die Umschalter S6 und S7 und über einen elektronischen Schalter 13 zu der Tonimpuls-Ausgabeeinheit, also dem Applikator 22. Dem elektronischen Schalter 13 ist eine Akustikverstärkerschaltung 17 und eine stereophone Klangquelle 18 zugeordnet. Die erzeugten Lichtimpulse, die durch die Akustikverstärkerschaltung 17 und Grund- und Endhelligkeit regulierbar sind, wirken auf zwei lichtempfindliche Widerstände, die in Reihe mit der stereophonen Klangquelle 18 geschaltet sind. Dabei wird das an die stereophonen Kopfhörer 28 (Fig. 4) gelangende Signal moduliert, wobei durch die opto-elektrische Kopplung ein störendes Schaltgeräusch vermieden wird.

Bei der an den digitalen Frequenzteiler 5 über den Umschalter S3 angeschlossenen Impulsformungseinheit 10 hat das am Eingang anliegende Rechtecksignal die gewünschte Frequenz, da der Frequenzteiler 5 die Teilung durch den Faktor 100 bereits vornimmt. Diese Impulsformungseinheit 10 weist ebenfalls eine Schmitt-Trigger-Invertierschaltung auf, die eine Einstellung des Puls-Pausenverhältnisses auf 50% vornimmt. Der Impulsformungseinheit 10 nachgeschaltet ist eine Umpolungsschaltung 14, der eine Verstärkerschaltung 19 zugeordnet ist. Die Umpolungsschaltung 14 besteht aus einem aus elektronischen Schaltern realisierten Umschalter, wobei je zwei elektronische Schalter das invertierte Signal und zwei elektronische Schalter das nicht-invertierte Signal empfangen. Daraus resultiert die Umschaltung der Stromflußrichtung der Verstärkerschaltung 19 in einem Tastverhältnis von 50%. Diese Verstärkerschaltung 19 liefert die Betriebsspannung für den elektromagnetischen Wandler 23. Dies kann eine Spule zur Erzeugung eines elektromagnetischen Feldes oder eine Vorrichtung zur Erzeugung einer mechanischen Vibration der gewünschten Frequenz sein.

Angeschlossen an das Gerät ist ein handelsüblicher stereophoner Kopfhörer 24 mit Hörmuscheln 28. An gegenüberliegenden Stellen der Außenseite des Kopfhörers 24 ist je ein Kugelgelenk 26 vorgesehen. An jedem Kugelgelenk 26 ist ein Halbbügel 25 schwenkbar angeordnet. Die freien Enden der beiden Halbbügel 25 sind gegeneinander gerichtet und an jedem Ende ist ein Leuchtkörper 27, beispielsweise ein Birnchen oder eine Leuchtdiode, vorgesehen. Die Stromzuführung erfolgt durch entsprechende Stromleiter, die im Rahmen eines jeden Halbbügels 25 verlegt und vor jedem Kugelgelenk 26 herausgeführt sind. Durch diese Konstruktion können die Leuchtkörper 27 leicht vor den Augen des Anwenders plaziert und entsprechend den unterschiedlichen anatomischen Gegebenheiten reguliert werden.

Elektroden 20 (Fig. 5) können in ähnlicher Form wie ein Ohrringteil im Sinne einer kleinen Schraubenzwinge ausgeführt sein. Eine solche läßt sich ebenfalls entsprechend der jeweiligen anatomischen Gegebenheiten des betreffenden Anwenders an einem beliebigen Ort der Ohrmuscheln befestigen und somit die an der Ohrmuschel befindlichen Akupunkturpunkte erreichen. Die Elektroden 20 sind an der Impulsformungseinheit 7 angeschlossen, die vom quarzstabilisierten Schwingkreis 1 über Frequenzteiler und den Umschalter S2 betätigt wird.

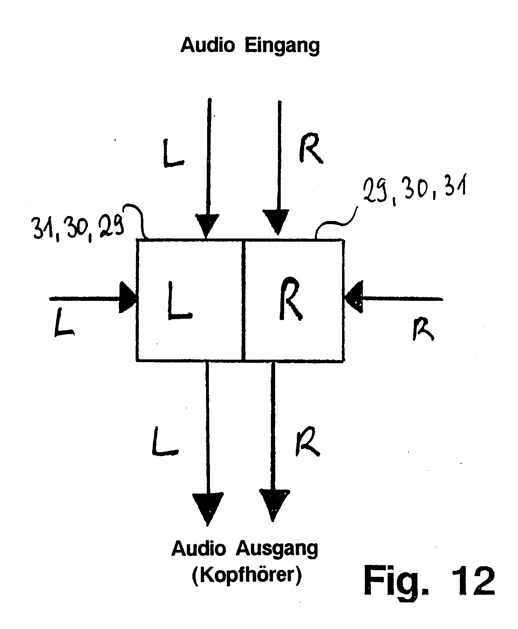

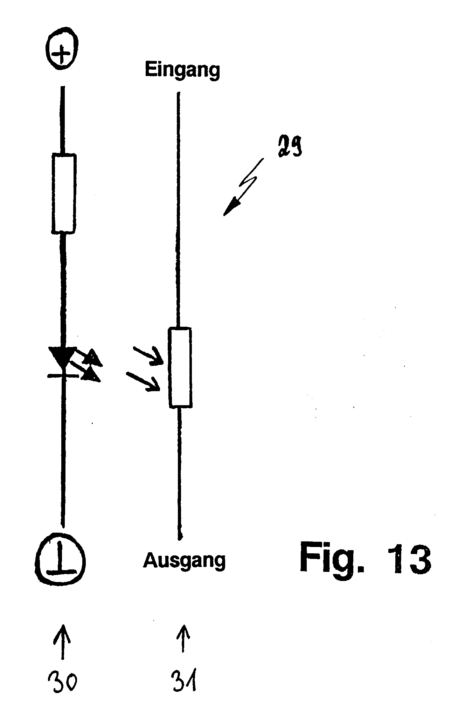

In Fig. 12 ist angedeutet, daß über Opto-Koppler 29 in einer in der Figur nicht gezeigten Klang-Wiedergabeeinheit erzeugte akustische Signale dem Tonimpuls-Applikator 22 zugeordnet werden. Gemäß der Darstellung in Fig. 13 weist der Opto-Koppler 29 eine LED 30 und einen Fotowiderstand 31 auf. Der Fotowiderstand 31 reagiert auf das Licht der LED 30 mit Widerstandserniedrigung. Da der Tonimpuls-Applikator 22, beispielsweise ein Kopfhörer, getrennt angesteuert werden muß, sind zwei Einheiten des Opto-Kopplers 29 erforderlich. Wesentlich ist dabei, daß die LED 30 so angeordnet ist, daß der Fotowiderstand 31 auf der lichtempfindlichen Fläche beleuchtet wird. Dies kann beispielsweise auf einer Platine geschehen. Vorzugsweise wird die LED 30 jedoch an der abstrahlenden Seite abgeflacht und mit transparentem Klebstoff auf den Fotowiderstand 31 geklebt. Zwei solcher Opto-Koppler 29 lassen sich beispielsweise auf einem 16-poligen IC-Sockel befestigen. Nach der Montage der entsprechenden Bauteile werden diese durch eine Kappe aus Kunststoff abgedeckt und mit vorzugsweise schwarz eingefärbtem Gießharz vergossen, so daß äußere Lichteinflüsse ausgeschaltet sind. Eine solche Baugruppe bzw. ein solches Modul ist in Fig. 12 - schematisch - dargestellt.

Die zuvor erörterte Schaltung des Opto-Kopplers benötigt keine eigene Stromversorgung. Darüber hinaus findet keine Beeinflussung des Audio-Signals durch den Impulsgenerator statt. Entsprechend treten keine Schaltgeräusche auf. Der in Rede stehende Opto-Koppler 29 liefert eine hohe Übertragungsqualität.

In den Figuren ist nicht dargestellt, daß der elektromagnetische Wandler 23 einen Wickelkörper aus Kunststoff und zwei darauf gewickelte, parallel laufende Drähte aufweisen kann. Bei diesen Drähten handelt es sich vorzugsweise um Co-Lack-Drähte. Die daraus resultierenden Spulen werden in weiter vorteilhafter Weise gegenläufig betrieben, so daß sich die von den Spulen ausgehenden Magnetfelder im wesentlichen aufheben. Solchen sich aufhebenden Magnetfeldern wird besondere biologische Wirkung zugeschrieben.

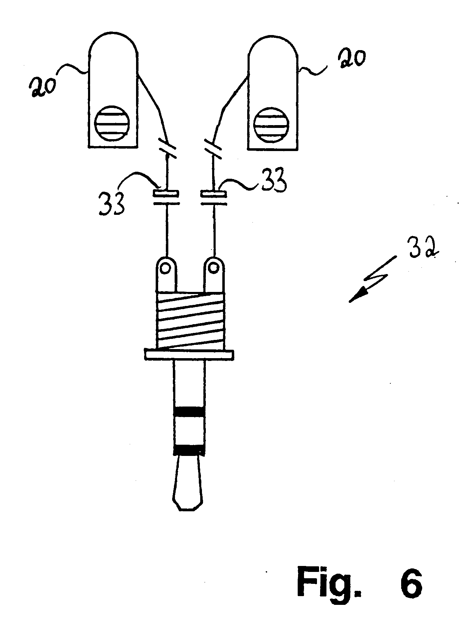

Gemäß der Darstellung in Fig. 6 sind die Elektroden 20 mittels eines vorzugsweise als Klinkenstecker ausgeführten Sägezahnimpuls-Steckers 32 an das erfindungsgemäße Gerät anschließbar.

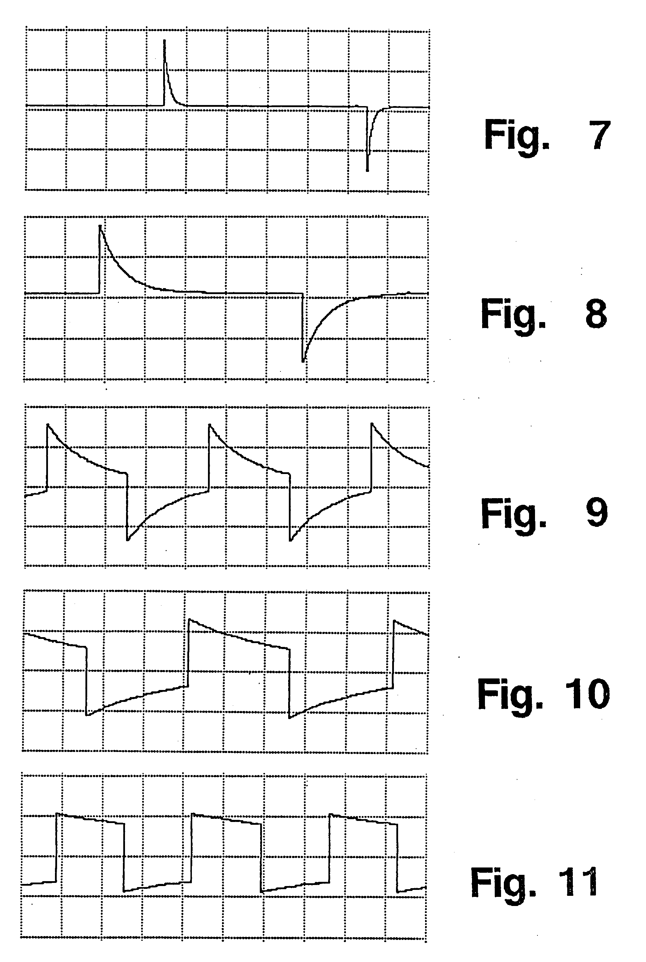

Zwischen Elektrode 20 und Klinkenstecker 32 ist jeweils ein vorzugsweise als Tantal-Elektrolytkondensator ausgebildeter Kondensator 33 zwischengeschaltet. Die Fig. 7 bis 11 zeigen die Beeinflussung des Impulsverlaufs durch die Sägezahnimpuls-Schaltung. Den Diagrammen der Fig. 7 bis 11 kann man entnehmen, daß die Beeinflussung der Wellenform gleitend von bipolaren, sehr kurzen, nadelartigen Sägezahnimpulsen im niedrigen Frequenzbereich über längere SZI abnimmt. Die Impulsfrequenzen betragen von Fig. 7 bis 11 0,2, 1, 5, 10 und 30 Hertz. Bei 30 Hertz findet nur noch eine unwesentliche Beeinflussung des vom Gerät erzeugten biphasischen Rechtecks statt.

Mittels der in Fig. 6 gezeigten Schaltung wird der "Gleichstromanteil" eliminiert, der sich bei biphasigen Rechteckimpulsen sehr niedriger Frequenzen bemerkbar macht (z.B. 0,2 Hertz entspricht 2,5 s Gleichstrom in jede Richtung). Außerdem resultiert aus der geänderten Wellenform ein differentes Obertonverhalten. Beim rechteckigen und dreieckigen Impulsverlauf liegen ausschließlich ungeradzahlige Obertöne, beim Sägezahnimpuls liegt ein ausgeglichenes Obertonspektrum vor.

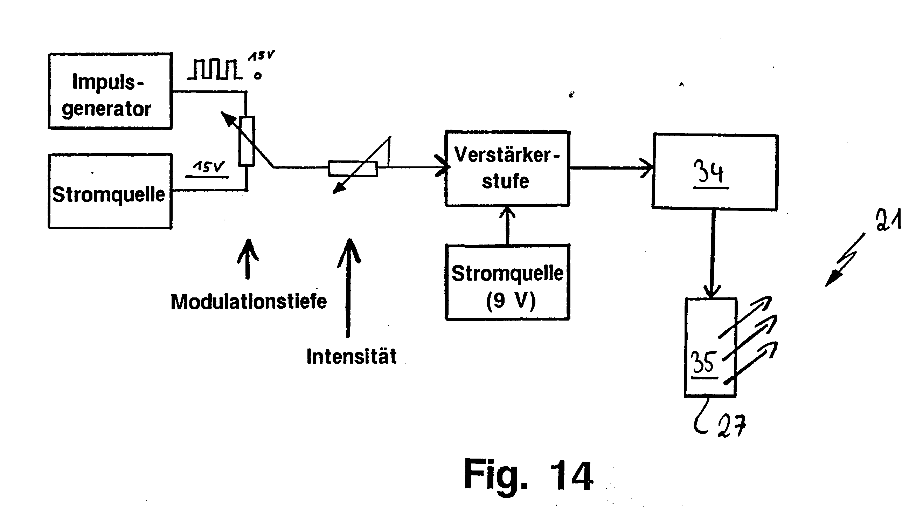

Fig. 14 zeigt schließlich in schematischer Darstellung eine als Lichtimpuls-Applikator 21 einsetzbare Fotonik. Dabei weist der Lichtimpuls-Applikator 21 als Leuchtkörper 27 eine mit einem über Gleichstrom betriebenen Starter 34 arbeitende Fotonik-Folie 35 auf. Der Lichtimpuls-Applikator 21 kann dabei so ausgeführt sein, daß je Auge eine Fotonik-Folie 35 vorgesehen ist. Der Lichtimpuls-Applikator 21 kann jedoch auch als Ganzfeldbrille ausgeführt sein. Die Fotonik-Folie 35 könnte sich dabei im Bereich beider Augen erstrecken.

Die Verwendung der Fotonik-Folie 35 hat den großen Vorteil, daß die Helligkeit, mit der die Fotonik-Folie 35 leuchtet, regelbar ist. Des weiteren ist die Modulationstiefe einstellbar.


## Patentansprüche

1. Gerät zur Hirnwellenstimulation mit einer Einrichtung zur Einstellung einer Frequenz und mit Impulsformungs- und Aufbereitungseinheiten zur Ausgabe akustischer, optischer, elektrischer und/oder elektromagnetischer bzw. mechanischer Impulssignale,
   **dadurch gekennzeichnet**, daß einem vorzugsweise quarzstabilisierten Schwingkreis (1) zur Frequenzeinstellung ein digitaler Frequenzteilerbaustein (2) nachgeschaltet ist, der über einen Umschalter (S1) mit einem programmierbaren digitalen Frequenzteiler (3) verbunden ist, daß der Frequenzteiler (3) eine definierte Frequenzeinstellung in Schritten bis zu 0,01 Hertz ermöglicht, daß dem Frequenzteiler (3) zwei parallel zueinander angeordnete digitale Frequenzteiler (4, 5) nachgeschaltet sind, daß die Frequenzteiler (4, 5) je zwei Ausgänge aufweisen, daß die Ausgänge über gekoppelte Umschalter (S2, S3) unterschiedliche Frequenzen an Impulsformungseinheiten (7, 8, 9, 10) für verschiedene Applikationsarten liefern und daß die Impulsformungseinheiten für angeschlossene Applikatoren Ausgangssignale erzeugen, die identische Frequenz aber unterschiedliche Impulsformen aufweisen.

2. Gerät nach Anspruch 1, dadurch gekennzeichnet, daß die Impulsformungseinheit (7) die Impulsform des Ausgangssignals einstellbar verändert, daß die Impulsformungseinheit (7) aus einem digitalen Frequenzteiler und einem durch Schalter (SX1 - SXn) programmierbaren digitalen Schieberegister (SR) besteht und als Hüllkurvengenerator das Schaltungsverhalten eines elektronischen Umpolungsschalters (11) definiert, welcher den in einer einstellbaren Stromquelle (15) erzeugten Strom den Elektroden-Applikatoren (20) zuordnet und daß Stromfluß und Stromflußrichtung durch die Stellung der Umschalter (SX1 - SXn) mit mittlerer Nullstellung bestimmt sind.

3. Gerät nach Anspruch 1, dadurch gekennzeichnet, daß die Impulsformungseinheit (8) als digitaler, in vorzugsweise 1%-Schritten einstellbarer Pulsbreitenmodulator mit zugeordneter Schmitt-Trigger-Invertierschaltung ausgelegt ist, deren invertierte und nicht-invertierte Ausgangssignale über die Umschalter (S4, S5) an zwei elektronische Schalter (12) gelangen, die, sofern sie durch das Steuersignal der Impulsformungseinheit (8) aktiviert sind, eine Lichtverstärkerschaltung (16) dem Lichtimpuls-Applikator (21) zuordnen.

4. Gerät nach Anspruch 1, dadurch gekennzeichnet, daß die Impulsformungseinheit (9) als digitaler, in vorzugsweise 1%-Schritten einstellbarer Pulsbreitenmodulator mit nachgeschalteter Schmitt-Trigger-Invertierschaltung ausgelegt ist, deren invertierte und nicht-invertierte Ausgangssignale über die Umschalter (S6, S7) an zwei elektronische Schalter (13) gelangen, die, sofern sie durch das Steuersignal der Impulsformungseinheit (9) aktiviert sind, eine Akustikverstärkerschaltung (17) dem Tonimpuls-Applikator (22) zuordnen, daß ggf. über Opto-Koppler (29) in einer externen Klang-Wiedergabeeinheit erzeugte akustische Signale dem Tonimpuls-Applikator (22) zugeordnet werden und daß der Opto-Koppler (29) vorzugsweise eine LED (30) und einen Fotowiderstand (31) aufweist, wobei der Fotowiderstand (31) auf das Licht der LED (30) mit Widerstandserniedrigung reagiert.

5. Gerät nach Anspruch 1, dadurch gekennzeichnet, daß der digitale Frequenzteiler (5) über einen Umschalter (S3) mit einer Impulsformungseinheit (10) verbunden ist, die als Schmitt-Trigger-Invertierschaltung ausgelegt ist und deren invertiertes und nicht-invertiertes Ausgangssignal einer elektronischen Umpolungsschaltung (14) zugeordnet ist, die eine Verstärkerschaltung (19) entsprechend der aus der Impulsformungseinheit (10) stammenden Steuersignale einem elektromagnetischen Wandler (23) zuordnet.

6. Gerät nach Anspruch 3 oder 4, dadurch gekennzeichnet, daß die Lichtverstärkerschaltung (16) so ausgeführt ist, daß sowohl die Grundhelligkeit als auch die Impulshelligkeit stufenlos über getrennte Einsteller regelbar ist und daß ggf. die Akustikverstärkerschaltung (17) so ausgeführt ist, daß sowohl die Grundlautstärke als auch die Impulslautstärke stufenlos über getrennte Einsteller regelbar ist.

7. Gerät nach Anspruch 5, dadurch gekennzeichnet, daß der elektromagnetische Wandler (23) ein auf den Anwender zur Einwirkung bringbares Magnetfeld definierter Frequenz erzeugt oder daß der elektromagnetische Wandler (23) einen Wickelkörper vorzugsweise aus Kunststoff und zwei darauf gewickelte, parallel laufende Drähte, vorzugsweise Cu-Lack-Drähte, aufweist, daß die daraus resultierenden Spulen gegenläufig betrieben werden und daß sich die von den Spulen ausgehenden Magnetfelder im wesentlichen aufheben oder daß der elektromagnetische Wandler (23) eine auf den Anwender zur Einwirkung bringbare mechanische Vibration erzeugt.

8. Gerät nach Anspruch 2 und ggf. einem der Ansprüche 3 bis 7, dadurch gekennzeichnet, daß Elektroden (20) durch die Stromquelle (15) betrieben werden, daß die Elektroden (20) mittels eines vorzugsweise als Klinkenstecker ausgeführten Sägezahnimpuls-Steckers (32) an das Gerät anschließbar sind und daß zwischen Elektrode (20) und Klinkenstecker jeweils ein vorzugsweise als Tantal-Elektrolytkondensator ausgebildeter Kondensator (33) zwischengeschaltet ist.

9. Gerät nach einem der Ansprüche 1 bis 8, dadurch gekennzeichnet, daß die Lichtimpuls- und Tonimpuls-Applikatoren (21, 22) aus einem handelsüblichen stereophonen Bügel-Kopfhörer (24, 25) bestehen, daß an den Seitenteilen des Kopfhörerbügels (24), einander gegenüberliegend, je ein vorzugsweise als Kugelgelenk (26) ausgebildetes Gelenk vorgesehen ist, an dem jeweils ein Halbbügel (25) schwenkbar angeordnet ist, daß die beiden einander zugekehrten freien Enden der Halbbügel (25) zur Aufnahme je eines Leuchtkörpers (27) mit einer entsprechenden Aufnahmefassung versehen sind, daß spannungsführende Anschlußkabel im Rahmen der Halbbügel (25) geführt und vor dem Kugelgelenk (26) herausgeführt sind und daß die je einen Leuchtkörper (27) tragenden Halbbügel (25) entsprechend dem Freiheitsgrad des Kugelgelenkes (26) stufenlos einstellbar und vor die Augen des Anwenders führbar und entsprechend dem Augenabstand genau einrichtbar sind.

10. Gerät nach einem der Ansprüche 1 bis 9, dadurch gekennzeichnet, daß der Lichtimpuls-Applikator (21) als Leuchtkörper (27) eine mit einem über Gleichstrom betriebenen Starter (34) arbeitende Fotonik-Folie (35) aufweist, daß die Helligkeit regelbar ist, mit der die Fotonik-Folie (35) leuchtet und daß die Modulationstiefe einstellbar ist.

## Claims

1. Apparatus for brain wave stimulation, comprising a device for setting a frequency and with units for shaping and preparing pulses for the output of acoustic, optical, electrical, and/or electromagnetic or mechanical pulse signals, characterised in that a digital frequency divider component (2) for frequency setting is connected downstream of an advantageously quartz-stabilised oscillator circuit (1), which component is connected to a programmable digital frequency divider (3) by means of a selector switch (S1); in that the frequency divider (3) makes a defined frequency setting in stages up to 0.01 Hertz possible; in that two digital frequency dividers (4,5), disposed parallel to one another, are connected downstream of the frequency divider (3); in that the frequency dividers (4,5) each comprise two outputs; in that the outputs supply different frequencies by means of coupled selector switches (S2,S3) to pulse-shaping units (7,8,9,10) for different types of application; and in that the pulse-shaping units generate output signals, which have the same frequency but different pulse shapes, for connected applicators.

2. Apparatus according to claim 1, characterised in that the pulse-shaping unit (7) adjustably alters the pulse shape of the output signal; in that the pulse-shaping unit (7) consists of a digital frequency divider and a digital shift register (SR) which can be programmed by means of switches (SX1 -SXn) and, as an envelope generator, defines the circuit behaviour of an electronic polarity reversing switch (11) which allocates the current generated in an adjustable current source (15) to the electrode applicators (20); and in that the current flow and the current flow direction are determined by the setting of the selector switch (SX1 -SXn) at a mean zero setting.

3. Apparatus according to claim 1, characterised in that the pulse-shaping unit (8) is designed as a digital pulse width modulator which is advantageously adjustable in 1% stages and comprises an associated Schmitt trigger inversion circuit, of which the inverted and non-inverted output signals pass, by means of the selector switches (S4, S5), to two electronic switches (12), which, provided they are activated by the control signal of the pulse-shaping unit (8), allocate a light-amplifying circuit (16) to the light pulse applicator (21).

4. Apparatus according to claim 1, characterised in that the pulse-shaping unit (9) is designed as a digital pulse width modulator which is advantageously adjustable in 1% stages and comprises a Schmitt trigger inversion circuit connected downstream, of which the inverted and non-inverted output signals pass, by means of the selector switches (S6, S7), to two electronic switches (13) which, provided they are activated by the control signal of the pulse-shaping unit (9), allocate an acoustic amplifier circuit (17) to the sound pulse applicator (22); in that acoustic signals, optionally generated via optocouplers (29) in an external sound reproduction unit, are optionally allocated to the sound pulse applicator (22); and in that the optocoupler (29) is advantageously an LED (30) and comprises a photoresistor (31), the photoresistor (31) reacting to the light of the LED (30) with a reduction in resistance.

5. Apparatus according to claim 1, characterised in that the digital frequency divider (5) is connected via a selector switch (S3) to a pulse-shaping unit (10), which is designed as a Schmitt trigger inversion circuit and to the inverted and non-inverted output signal of which there is allocated an electronic polarity reversal circuit (14), which allocates an amplifier circuit (19) to an electromagnetic transducer (23) in a manner dependent on the control signals originating from the pulse shaping unit (10).

6. Apparatus according to claim 3 or claim 4, characterised in that the light-amplifier circuit (16) is formed such that both the amount of basic light and the amount of pulse light can be adjusted continuously by means of separate adjusting devices; and in that the acoustic amplifier signal (17) is optionally formed such that both the basic sound level and the pulse sound level can be adjusted continuously by means of separate adjusting devices.

7. Apparatus according to claim 5, characterised in that the electromagnetic transducer (23) generates a magnetic field which can be made to act on the user and is of a defined frequency; or in that the electromagnetic convertor (23) comprises a wound element, advantageously of plastics material, and two parallel wires, advantageously lacquered copper wires, wound thereon; in that the resultant coils are operated in opposite directions; and in that the magnetic fields radiating from the coils substantially cancel one another out; or in that the electromagnetic transducer (23) generates a mechanical vibration which can be made to act on the user.

8. Apparatus according to claim 2 and optionally to any one of claims 3 to 7, characterised in that electrodes (20) are operated by the current source (15); in that the electrodes (20) can be connected to the apparatus by means of a saw-tooth pulse plug (32) which is advantageously formed as a jack plug; and in that a capacitor (33), advantageously formed as a tantalum electrolytic capacitor, is connected between the electrode (20) and the jack plug in each case.

9. Apparatus according to one of claims 1 to 8, characterised in that the light pulse and sound pulse applicators (21,22) consist of a commercially available stereophonic arch-shaped headphones (24,25); in that joints, advantageously formed as ball-and-socket joints (26), are provided on the side pieces of the arch-shaped headphones in each case, on which joints a semi-arch (25) is pivotably disposed in each case;

in that the two facing free ends of the semi-arch (25) are provided with a suitable accommodating holder for accommodating a light element (27) in each case; in that current-conducting connection cables are conducted into the frame of the semi-arch (25) and conducted out in front of the ball-and-socket joint (26); and in that the semi-arches (25), which each support one light element (27), can be adjusted continuously in accordance with the degree of movement of the ball-and-socket joint (26) and can be brought in front of the eyes of the user and adjusted exactly corresponding to the distance between the eyes.

10. Apparatus according to one of claims 1 to 9, characterised in that the light pulse applicator (21) comprises, as a light element (27), a photonik film (35) working with a starter (34) operated by means of direct current flow; in that the amount of light emitted by the photonik film (35) is adjustable; and in that the depth of modulation is adjustable.

## Revendications

1. Appareil de stimulation des ondes cérébrales, comprenant un dispositif pour le réglage de la fréquence et des unités de formation des impulsions et de traitement pour la sortie d'impulsions acoustiques, optiques, électriques et/ou électromagnétiques ou mécaniques, **caractérisé en ce** qu'un circuit oscillant (1) pour le réglage de la fréquence, de préférence stabilisé par cristal, est suivi d'un composant diviseur de fréquence numérique (2) qui, par l'intermédiaire d'un commutateur (S1), est couplé avec un diviseur de fréquence numérique (3), que le diviseur de fréquence (3) permet un réglage de fréquence défini par pas allant jusqu'à 0,01 hertz, que le diviseur de fréquence (3) est suivi de deux diviseurs de fréquence numériques (4, 5) montés en parallèle, que les diviseurs de fréquence (4, 5) présentent chacun deux sorties, que les sorties délivrent, par l'intermédiaire de commutateurs couplés (S2, S3), des fréquences différentes à des unités formatrices d'impulsions (7, 8, 9, 10) pour différents types d'applications, et que les unités formatrices d'impulsions génèrent pour des applicateurs raccordés, des signaux de sortie qui présentent une fréquence identique mais des formes d'impulsion différentes.

2. Appareil selon la revendication 1, caractérisé en ce que l'unité formatrice d'impulsions (7) modifie de manière réglable la forme d'impulsion du signal de sortie, que l'unité formatrice d'impulsions (7) se compose d'un diviseur de fréquence numérique et d'un registre de décalage numérique (SR) pouvant être programmé par des commutateurs (SX1 à SXn) et qu'elle définit, en tant que générateur d'enveloppantes le comportement de commutation d'un inverseur de polarité électronique (11) qui associe le courant produit dans une source de courant (15) réglable aux applicateurs d'électrodes (20), et que la conduction de courant et la direction de conduction de courant sont déterminées par la position des commutateurs (SX1 à SXn) à position zéro médiane.

3. Appareil selon la revendication 1, caractérisé en ce que l'unité formatrice d'impulsions (8) est réalisée sous la forme d'un modulateur d'impulsions en durée numérique réglable de préférence par pas de 1 % avec circuit d'inversion à bascule de Schmitt associé dont les signaux de sortie inversés et non-inversés sont transmis par l'intermédiaire des commutateurs (S4, S5) à deux commutateurs électroniques (12) qui, dans la mesure où ils sont activés par le signal de commande de l'unité formatrice d'impulsions (8), associent à l'applicateur d'impulsions lumineuses (21) un circuit d'amplification de lumière (16).

4. Appareil selon la revendication 1, caractérisé en ce que l'unité formatrice d'impulsions (9) est réalisée sous la forme d'un modulateur d'impulsions en durée réglable de préférence par pas de 1 % avec circuit d'inversion à bascule de Schmitt associé dont les signaux de sortie inversés et non-inversés sont transmis par l'intermédiaire des commutateurs (S6, S7) à deux commutateurs électroniques (13) qui, dans la mesure où ils sont activés par le signal de commande de l'unité formatrice d'impulsions (9), associent à l'applicateur d'impulsions sonores (22) un circuit d'amplification acoustique (17), que des signaux acoustiques générés éventuellement par l'intermédiaire de coupleurs optoélectroniques (29) dans une unité de reproduction de sons externe sont associés à l'applicateur d'impulsions sonores (22), et que le coupleur optoélectronique (29) comporte de préférence une DEL (30) et une photorésistance (31), la photorésistance (31) réagissant à la lumière de la DEL (30) par une diminution de la résistance.

5. Appareil selon la revendication 1, caractérisé en ce que le diviseur de fréquence numérique (5) est couplé par l'intermédiaire d'un commutateur (S3) avec une unité formatrice d'impulsions (10) qui est conformée en circuit d'inversion à bascule de Schmitt et dont le signal de sortie inversé et non-inversé est associé à un circuit d'inversion de polarité électronique (14) qui associe un circuit d'amplification (19) à un convertisseur électromagnétique (23) en fonction des signaux de commande provenant de l'unité formatrice d'impulsions (10).

6. Appareil selon l'une des revendications 3 ou 4, caractérisé en ce que le circuit d'amplification de lumière (16) est réalisé de telle façon que la luminosité du fond aussi bien que la luminosité des impulsions peuvent être réglées en continu par l'intermédiaire de régulateurs séparés, et que le circuit d'amplification acoustique (17) est réalisé, le cas échéant, de telle façon que l'intensité sonore du fond aussi bien que l'intensité sonore des impulsions peuvent être réglées en continu par l'intermédiaire de régulateurs séparés.

7. Appareil selon la revendication 5, caractérisé en ce que le convertisseur électromagnétique (23) génère un champ magnétique d'une fréquence définie pouvant être appliqué à l'utilisateur ou que le convertisseur électromagnétique (23) comprend un corps de bobinage de préférence en matière plastique sur lequel sont enroulés deux fils parallèles, de préférence des fils en cuivre émaillé, que les bobines qui en résultent fonctionnent en sens inverse et que les champs magnétiques partant desdites bobines s'annulent sensiblement ou que le convertisseur électromagnétique (23) génère une vibration mécanique pouvant être appliquée à l'utilisateur.

8. Appareil selon la revendication 2 et éventuellement selon l'une des revendications 3 à 7, caractérisé en ce que des électrodes (20) sont alimentées par la source de courant (15), que les électrodes (20) peuvent être reliées à l'appareil au moyen d'un connecteur (32) à impulsions en dent de scie, de préférence réalisé sous forme de jack, et qu'entre l'électrode (20) et le jack est intercalé respectivement un condensateur (33), de préférence réalisé sous la forme d'un condensateur électrochimique au tantale.

9. Appareil selon l'une des revendications 1 à 8, caractérisé en ce que les applicateurs d'impulsions lumineuses et d'impulsions sonores (21, 22) sont constitués par un casque serre-tête stéréophonique (24, 25) disponible dans le commerce, que sur les éléments latéraux de l'étrier du casque (24) est prévue, respectivement en face l'une de l'autre, une articulation, de préférence conformée en rotule (26) sur laquelle est monté à chaque fois de manière pivotante un demi-étrier (25), que les deux extrémités libres des demi-étriers (25) tournées l'une vers l'autre sont munies d'une douille correspondante pour la mise en place de respectivement un corps luminescent (27), que des câbles de raccordement sous tension sont guidés dans le cadre des demi-étriers (25) et sortis en amont de la rotule (26), et que les demi-étriers (25) portant chacun un corps luminescent (27) peuvent être réglés conformément au degré de liberté de la rotule (26) et amenés devant les yeux de l'utilisateur et réglés avec précision en fonction de la distance interoculaire.

10. Appareil selon l'une des revendications 1 à 9, caractérisé en ce que l'applicateur d'impulsions lumineuses (21) comporte comme corps luminescent (27) une feuille photonique (35) fonctionnant à l'aide d'un starter (34) alimenté en courant continu, que la luminosité de la feuille photonique (35) est réglable, et que la profondeur de la modulation est réglable.

**Fig. 1**

EP 0 502 919 B1

# Fig. 2

EP 0 502 919 B1

# Fig. 3

EP 0 502 919 B1

# Fig. 5

20

# Fig. 4

25

26

27

24

28

25

26

**Fig. 6**

EP 0 502 919 B1

Fig. 7

Fig. 8

Fig. 9

Fig. 10

Fig. 11

16

**Audio Eingang**

L  R

31,30,29   29,30,31

L | L | R | R

L   R

**Audio Ausgang**
**(Kopfhörer)**

# Fig. 12

Eingang

29

Ausgang

# Fig. 13

30

31

Impulsgenerator

Stromquelle

15V

Verstärkerstufe

34

Stromquelle
(9 V)

35

Modulationstiefe

Intensität

# Fig. 14